# EUROPÄISCHE PATENTSCHRIFT

(11) **EP 3 310 423 B1**
(45) Veröffentlichungstag und Bekanntmachung des Hinweises auf die Patenterteilung: **26.09.2018**
(21) Anmeldenummer: 16756604.1
(22) Anmeldetag: 02.08.2016
(51) Int. Cl.: A61M 25/02

(54) **SET ZUR FIXIERUNG EINES KATHETERSCHLAUCHS**
KIT FOR FIXING A CATHETER HOSE
ENSEMBLE POUR LA FIXATION D'UN TUBE DE CATHÉTER

(30) Priorität: 06.08.2015 DE 102015215058
(43) Veröffentlichungstag der Anmeldung: 25.04.2018
(73) Patentinhaber: Novo Klinik-Service GmbH, 50127 Bergheim (DE)
(72) Erfinder: ERASLAN, Oliver, 50939 Köln (DE); KARI, Veronika, 52388 Nörvenich (DE); LANG, Benjamin, 50170 Kerpen (DE)
(74) Vertreter: Maikowski & Ninnemann Patentanwälte Partnerschaft mbB
(86) Internationale Anmeldenummer: PCT/EP2016/068433
(87) Internationale Veröffentlichungsnummer: WO 2017/021409

(56) Entgegenhaltungen:
- WO-A1-01/53737
- WO-A1-2014/169741
- US-A- 4 867 154
- US-A- 5 019 050
- US-A- 5 147 322
- US-A- 5 304 146
- US-A- 6 015 119

## Beschreibung

Die vorliegende Erfindung betrifft ein Set zur Fixierung eines Katheterschlauchs gemäß dem Oberbegriff des Anspruchs 1 und ein Verfahren zur Fixierung eines Katheterschlauchs gemäß dem Oberbegriff des Anspruchs 9.

Ein solches Set kann beispielsweise zur Fixierung eines Katheterschlauchs im Rahmen der Extrakorporalen Membranoxygenierung (ECMO) eingesetzt werden. Dabei ist es gleichermaßen für Erwachsene, Jugendliche und Schulkinder zur Sicherung und Zugentlastung eines Katheters an der jeweiligen Kathetereinstichstelle geeignet.

Gemäß dem Stand der Technik werden ECMO-Katheterschlauchsysteme mit gewöhnlichen Heftpflastern und/oder durch zweckentfremdete Fixierbänder in der Nähe der Kathetereinstichstelle fixiert.

Durch die Fixierung von ECMO-Katheterschlauchsystemen mit derartigen gewöhnlichen Heftpflastern bzw. Fixierbändern, die nicht für diesen Zweck vorgesehen sind, entstehen beim betroffenen Patienten innerhalb kürzester Zeit Haut- und Weichteilschädigungen. Die bislang eingesetzten Fixierungsmaterialien können aufgrund ihrer Materialbeschaffenheit keine ausreichende Sicherung und damit auch keine adäquate Zugentlastung eines ECMO-Katheterschlauchsystems gewährleisten. Daher besteht bei den bislang eingesetzten Fixierungsverfahren ein erhebliches Risiko einer Dislokation des zu fixierenden ECMO-Katheterschlauchsystems. Eine solche Dislokation kann jedoch unmittelbar zum Tode des betroffenen Patienten führen und ist daher unbedingt zu vermeiden.

Aus dem Stand der Technik ist auch ein Kopfhalter-Set zur Fixierung eines ECMO-Katheterschlauchsystems bekannt. Dieses Kopfhalter-Set besteht aus einem Katheter-Fixierband, das in Stirnhöhe in Kopfumfangsrichtung um den Kopf eines Patienten gelegt wird. Zur Fixierung dieses Fixierbandes wird ein zusätzliches Kopfband angelegt, das einerseits mit dem Fixierband verbunden ist und andererseits über ein Wangenpflaster an einer Wange des betroffenen Patienten fixiert wird. Dabei wird das Kopfband über den Scheitel des Patienten geführt. Ein solches zusätzliches Kopfband wird jedoch häufig als unkomfortabel empfunden, da es im Scheitelbereich des Kopfes einen Druck auf den Patienten ausübt. Zudem wird das Verrutschen der Fixierung durch das Kopfband nicht effektiv verhindert.

Die US 4,861,154 A beschreibt ein rahmenartig geschwungenes Band, das mittels dreier Pflaster auf der Haut eines Patienten befestigt wird. Dazu werden die Pflaster auf die Haut des Patienten aufgeklebt. Die Pflaster können auf ihrer Oberseite einen Klettbereich aufweisen. Über diesen Klettbereich wird dann ein entsprechendes Gegenstück unter Ausbildung eines Klettverschlusses fixiert, wobei das Gegenstück das rahmenartig geschwungene Band fixiert. Dieses Band dient dazu, einen Endotrachealtubus in Position zu halten.

Die US 5,019,050 A beschreibt ein Fixierband zur Befestigung einer Infusionsnadel oder eines ähnlichen Gegenstands. Dieses Fixierband wird fest um eine Extremität eines Patienten herumgeführt und an sich selbst befestigt.

Die US 6,015,119 A und die WO 01/53737 A1 beschreiben jeweils eine Befestigungseinheit, die der Befestigung eines Schlauchs dient und mittels eines Klettverschlusses an einer Ankereinheit festgelegt wird.

Die US 5,304,146 A beschreibt ein auf der Haut eines Patienten zu befestigendes Pflaster, das der Fixierung eines Schlauches durch zwei Stege, die integraler Bestandteil des Pflasters sind, dient. Wenn eine Zugkraft auf den Schlauch ausgeübt wird, überträgt sich diese unmittelbar auf das Pflaster und somit die Haut des Patienten.

Die US 5,147,322 A beschreibt ebenfalls ein auf der Haut eines Patienten zu befestigendes Pflaster, bei dem ein Schlauch durch eine Haltelasche gehalten wird, die ein integraler Bestandteil des Pflasters ist.

Die WO 2014/169741 A1 beschreibt ein Fixierband zum Fixieren eines Katheters. Dieses Fixierband wird eng um das Bein eines Patienten geschlungen. Dabei weist es einen geschlitzten Befestigungsstreifen auf, durch den ein Belüftungsventilanschluss des Katheters geführt werden kann.

Die WO 2004/091406 A1 betrifft einen Halter für einen Übertrager (Transducer). Dieser Halter weist drei voneinander getrennte Klettschlaufen auf, die jeweils zur Aufnahme eines einzigen Transducers vorgesehen sind.

Die US 2002/0165495 A1 beschreibt ein Befestigungsband, das auf seiner Innenseite mit einer rutschfesten Oberfläche versehen ist. Auf seiner Außenseite kann ein einziges Lumen eines Katheters durch eine auf der Außenseite des Bandes fixierte Schlaufe gehalten werden.

Der vorliegenden Erfindung liegt die Aufgabe zugrunde, eine Fixierung eines doppellumigen Katheterschlauchs zu ermöglichen, die eine Zugentlastung an der Einstichstelle des betreffenden Katheters bietet und somit das Risiko einer versehentlichen Dislokation des Katheters minimiert, dabei zumindest ebenso sicher wie die aus dem Stand der Technik bekannten Lösungen ist, gleichzeitig aber einen höheren Patientenkomfort bietet.

Diese Aufgabe wird mit einem Set zur Fixierung eines Katheterschlauchs mit den Merkmalen des Anspruchs 1 gelöst. Ein solches Set weist ein erstes Pflaster, ein zweites Pflaster, ein erstes Fixierelement und ein Fixierband auf. Mit diesen vier Bestandteilen ist eine sichere, funktionale und hautfreundliche Fixierung eines Katheterschlauchs, beispielsweise für ein ECMO-Katheterschlauchsystem, möglich.

Das erste Pflaster weist eine erste Seite und eine der ersten Seite gegenüberliegende zweite Seite auf. Die erste Seite weist einen Klebstoff auf, mittels dessen das Pflaster auf der Haut eines Patienten fixiert werden kann. Derartige Klebstoffbeschichtungen einer Seite eines Pflasters sind aus dem Stand der Technik hinlänglich bekannt. Die Klebstoffbeschichtung wird üblicherweise mit einem Schutzfilm überdeckt, der unmittelbar vor Gebrauch des Pflasters abgezogen wird. Die zweite Seite des ersten Pflasters ist mit einem Klettfunktionsbereich ausgestattet. Ein solcher Klettfunktionsbereich dient dazu, mit einem anderen Klettfunktionsbereich einen Klettverschluss ausbilden zu können. Der Klettfunktionsbereich kann beispielsweise ein Haken- oder Pilzkopfband (harter Klettfunktionsbereich) sein. Alternativ wäre es auch möglich, dass der Klettfunktionsbereich ein Flauschband, ein Veloursband, ein Filzband oder eine Wirkware wäre (weicher Klettfunktionsbereich).

Das zweite Pflaster entspricht von seinem grundsätzlichen Aufbau her dem ersten Pflaster. So weist auch das zweite Pflaster eine erste Seite auf, die mit einem Klebstoff versehen ist, mit dem das zweite Pflaster auf der Haut des Patienten angebracht werden kann. Ferner ist der ersten Seite gegenüberliegend eine zweite Seite angeordnet, die einen Klettfunktionsbereich aufweist.

Vorzugsweise handelt es sich bei dem Klettfunktionsbereich in das ersten Pflasters und/oder des zweiten Pflasters um einen harten Klettfunktionsbereich.

Das erste Fixierelement dient zum Fixieren zweier Lumina eines doppellumigen Katheterschlauchs an dem ersten Pflaster, indem das erste Fixierelement eine durch einen Klettverschluss verschlossene Schlaufe um die beiden Lumina bildet. Zu diesem Zweck ist die gesamte nach außen weisende Oberfläche des ersten Fixierelements als Klettfunktionsbereich ausgestaltet, um ein besonders einfaches und positionsunabhängiges Fixieren des ersten Fixierelements an dem ersten Pflaster zu ermöglichen. Dabei bildet der Klettfunktionsbereich des ersten Fixierelements mit dem Klettfunktionsbereich des ersten Pflasters einen Klettverschluss aus.

Das Fixierband dient schließlich dazu, um einen Körperteil des Patienten, beispielsweise um einen Kopf entlang des Kopfumfangs, herumgeführt zu werden. Dabei kann das Fixierband stufenlos an die Größe des betreffenden Körperteils, beispielsweise an den Kopfumfang, des Patienten angepasst werden. Bei Bedarf kann das Fixierband auch entsprechend den jeweiligen Gegebenheiten bzw. Erfordernissen individuell gekürzt werden. Dies ist aufgrund der vorzugsweise eingesetzten Materialien besonders einfach möglich. Ferner wird das Fixierband an dem Körperteil des Patienten mittels des zweiten Pflasters fixiert. Um diesbezüglich eine einfache Fixierung des Fixierbandes an dem zweiten Pflaster zu ermöglichen, weist das Fixierband an einer Oberflächenseite, die beim bestimmungsgemäßen Gebrauch des Set zum Körperteil des Patienten hin orientiert ist, einen Klettfunktionsbereich auf, der mit dem Klettfunktionsbereich des zweiten Pflasters einen Klettverschluss ausbildet. Beispielsweise kann die gesamte Oberfläche des Fixierbandes, die beim bestimmungsgemäßen Gebrauch zum Kopf des Patienten hin orientiert ist, als Klettfunktionsbereich ausgestaltet sein.

Das Fixierband weist ein zweites Fixierelement auf, das zum Fixieren eines ersten Lumens des doppellumigen Katheterschlauchs an dem Fixierband dient. Dieses zweite Fixierelement ist insbesondere auf einer Außenseite des Fixierbandes, die beim bestimmungsgemäßen Gebrauch des Fixierbandes vom Körperteil des Patienten weg weist, angeordnet.

Das Fixierband weist außerdem ein drittes Fixierelement auf, das zum Fixieren eines zweiten Lumens des doppellumigen Katheterschlauchs an dem Fixierband vorgesehen und eingerichtet ist. Das dritte Fixierelement ist unmittelbar benachbart zu dem zweiten Fixierelement angeordnet. So ist es auf besonders einfache Art und Weise möglich, einen doppellumigen Katheterschlauch mit dem beanspruchten Set zu fixieren. Denn dann werden ein erstes Lumen des doppellumigen Katheterschlauchs von dem zweiten Fixierelement und ein zweites Lumen des doppellumigen Katheterschlauchs von dem dritten Fixierelement an dem Fixierband gehalten. Das zweite Fixierelement und das dritte Fixierelement sind derart dimensioniert, dass sie zur Aufnahme eines Lumens eines doppellumigen Katheterschlauchs besonders gut geeignet sind. Dann lassen sich zwei Lumina eines doppellumigen Katheterschlauchs (in Richtung zur Einstichstelle des betreffenden Katheters) zunächst getrennt durch das zweite und dritte Fixierelement führen, um dann gemeinsam durch das erste Fixierelement geführt zu werden, was einen optimierten Verlauf der einzelnen Lumina eines derartigen doppellumigen Katheterschlauchs ermöglicht.

Das vorliegend beanspruchte Set zur Fixierung eines Katheterschlauchs eignet sich insbesondere dazu, einen ECMO-Katheterschlauch zu fixieren, und zwar insbesondere an einem Kopf eines Patienten. Das Fixierband wird dann entlang des Kopfumfangs um den Kopf des Patienten herum gelegt und dort fixiert.

Anders als bei den aus dem Stand der Technik bekannten Lösungen ist es bei dem vorliegend beanspruchten Set zur Fixierung eines Katheterschlauchs nicht mehr erforderlich, eine zusätzliche Fixierung, beispielsweise in Form eines Kopfbandes, welches über den Scheitel eines Patienten geführt wird, vorzusehen. Vielmehr erfolgt eine Fixierung auf sichere und funktionale Art und Weise mittels des ersten Pflasters und des zweiten Pflasters, wobei der zu fixierende Katheterschlauch mittels des ersten und des zweiten Fixierelements selbst eine Verbindung zwischen dem Fixierband und dem ersten Pflaster ausbildet. In einer Variante weist das Set gerade kein Kopfband, das über den Scheitel eines Patienten zu führen ist, auf.

In einer Variante sind das erste Pflaster und das zweite Pflaster baugleich ausgestaltet. Beispielsweise können sie eine dreieckige Grundform aufweisen, wobei der Klettfunktionsbereich typischerweise nicht die gesamte Grundfläche des ersten Pflasters bzw. des zweiten Pflasters einnimmt. So kann beispielsweise ein unterschiedlich breiter, umlaufender Rand um den Klettfunktionsbereich vorgesehen sein. In einer Variante ist es möglich, dass der umlaufende Rand gleichmäßig dick ausgestaltet ist. Ferner kann der Klettfunktionsbereich die Grundform der Fläche des ersten Pflasters bzw. des zweiten Pflasters aufweisen oder aber eine hiervon abweichende Gestalt haben. Entscheidend ist letztlich nur, dass der Klettfunktionsbereich eine ausreichende Größe hat, um mit dem jeweiligen Gegenstück einen Klettverschluss mit einer ausreichend großen Haltekraft ausbilden zu können.

In einer Variante sind das erste Fixierelement und/oder das zweite Fixierelement als flächig erstrecktes Band ausgestaltet. Dieses Band weist in einer Variante eine erste Seite auf, die einen harten Klettfunktionsbereich aufweist. Ferner weist das Band in dieser Variante eine der ersten Seite gegenüberliegende zweite Seite auf, die einen weichen Klettfunktionsbereich aufweist. Der harte Klettfunktionsbereich und der weiche Klettfunktionsbereich bilden dabei einen Klettverschluss aus. Damit ist es möglich, das flächig erstreckte Band unter Ausbildung eines Klettverschlusses zusammenzurollen oder zusammenzulegen. Dies ermöglicht eine sichere Aufnahme eines Katheterschlauchs im Inneren des zusammengerollten oder zusammengelegten Bandes, sodass die erste Seite mit dem harten Klettfunktionsbereich nach innen zum Katheterschlauch hin orientiert ist, während die zweite Seite mit dem weichen Klettfunktionsbereich nach außen hin orientiert ist. Dies sichert einem Patienten einen hohen Tragekomfort zu und ermöglicht die Ausbildung einer Fixierschlaufe durch das erste Fixierelement und/oder das zweite Fixierelement, ohne dass hierfür weitere Elemente (wie etwa zusätzliche Verschlusselemente) notwendig wären.

In einer weiteren Variante weist das Band eine erste Seite auf, die einen weichen Klettfunktionsbereich aufweist. Ferner weist das Band in dieser Variante eine der ersten Seite gegenüberliegende zweite Seite auf, die ebenfalls einen weichen Klettfunktionsbereich aufweist. In dieser Variante können beispielsweise flächig erstreckte Bänder verwendet werden, die vollständig aus einem Schaumstoff bestehen. Diese Ausgestaltungsvariante eignet sich insbesondere für das erste Fixierelement, das im Wangenbereich eingesetzt wird.

In einer Variante ist das zweite Fixierelement fest mit dem Fixierband verbunden. Diese Verbindung kann beispielsweise durch ein Vernähen oder ein Verschweißen realisiert sein. Durch eine derartige feste Verbindung wird das zweite Fixierelement dauerhaft fest am Fixierband gehalten, sodass ein Katheterschlauch, der durch das zweite Fixierelement fixiert wird, automatisch fest mit dem Fixierband verbunden ist. Die feste Verbindung des Fixierelements mit dem Fixierband kann beispielsweise in einem mittigen Bereich des Fixierelements ausgebildet sein. Auf diese Weise lassen sich dann die beiden von dem Bereich der festen Verbindung abstehenden freien Enden besonders leicht zu einer Fixierschlaufe zusammenlegen, in deren Innenbereich der zu fixierende Katheterschlauch aufgenommen werden kann.

Um für einen besonders sicheren Halt eines zu fixierenden Katheterschlauchs innerhalb des ersten Fixierelements und/oder des zweiten Fixierelements zu sorgen, weisen das erste Fixierelement und/oder das zweite Fixierelement in einer Variante einen Haftbereich auf. Dieser Haftbereich ist dabei in einem Abschnitt angeordnet, der im bestimmungsgemäßen Einsatz des Sets in Kontakt mit dem zu fixierenden Katheterschlauch steht. Durch den Haftbereich wird dann eine zusätzliche Haltekraft zwischen dem ersten Fixierelement und/oder dem zweiten Fixierelement auf der einen Seite und dem zu fixierenden Katheterschlauch auf der anderen Seite aufgebaut. Auf diese Weise ist eine rutschfeste Verbindung des zu fixierenden Katheterschlauchs mit dem ersten Fixierelement und/oder dem zweiten Fixierelement und damit dem gesamten Set möglich.

Der Haftbereich kann beispielsweise durch ein mit einem Klebstoff versehenes netzartiges Element ausgebildet sein oder durch eine Gummierung, welche beispielsweise direkt auf das Fixierelement aufgebracht ist. In einer Variante ist der Haftbereich in einem mittigen Abschnitt des ersten Fixierelements und/oder des zweiten Fixierelements angeordnet, beispielsweise im Verbindungsbereich zwischen dem zweiten Fixierelement und dem Fixierband.

In einer Variante ist das dritte Fixierelement in gleicher Art und Weise wie das zweite Fixierelement ausgestaltet, einschließlich der bereits zuvor erwähnten möglichen Ausgestaltungsvarianten wie etwa des Haftbereichs.

In einer weiteren Variante weist das Fixierband an einem seiner Enden einen Klettfunktionsbereich auf, der mit einem Oberflächenabschnitt des Fixierbandes einen Klettverschluss bildet. Dadurch ist es möglich, das Fixierband in der gewünschten Länge um einen Körperteil eines Patienten, insbesondere um einen Kopf eines Patienten, herum zu legen und dabei die Größe des Fixierbandes an die Größe des Körperteils anzupassen. Ferner wird durch einen derartigen Klettverschluss ein unbeabsichtigtes Lösen des Fixierbandes verhindert. Der Klettverschluss kann beispielsweise durch Vernähen oder Verschweißen auf einer Außenseite des Fixierbandes angebracht sein. Alternativ ist es auch möglich, den Klettverschluss auf einer Innenseite des Fixierbandes anzubringen (sogenannte Back-to-back-Anordnung).

In einer weiteren Variante weist das Fixierband eine einheitlich ausgebildete Oberfläche auf einer ersten Seite und/oder auf einer der ersten Seite gegenüberliegenden zweiten Seite auf, soweit man von dem zweiten Fixierelement und dem zuvor erwähnten optionalen dritten Fixierelement sowie dem zuvor erwähnten optionalen Klettfunktionsbereich an einem seiner Enden absieht. Diese einheitlich ausgebildete Oberfläche lässt sich besonders einfach herstellen und gewährleistet einen hohen Tragekomfort für den Patienten sowie ein einfaches Festlegen des Fixierbandes an dem zweiten Pflaster. Bei einer derartig einheitlich ausgestalteten Oberfläche fungiert die gesamte Oberfläche als Klettfunktionsbereich, so dass nicht auf die Positionierung des Fixierbandes geachtet werden muss, wenn ein Klettverschluss (beispielsweise zum Schließen des Funktionsbandes oder zum Festlegen des Funktionsbandes mithilfe des zweiten Pflasters) ausgebildet werden soll.

Eine derart einheitlich ausgestaltete Oberfläche kann in einer Variante - unabhängig davon, ob sie beim Fixierband vorgesehen ist oder nicht - beim ersten Fixierelement vorgesehen sein.

In einer Variante weist das Fixierband eine veloursartige Oberfläche auf. Es hat sich gezeigt, dass Baumwolle ein geeignetes Material ist, um eine solche Oberfläche bereitzustellen. Harte Klettfunktionsbereiche können mit einer derartigen veloursartigen Oberfläche als weichem Klettfunktionsbereich problemlos wechselwirken, um einen Klettverschluss auszubilden. Zwischen der veloursartigen Oberfläche kann in einer Variante ein Schaumstoff, wie etwa ein atmungsaktiver Schaum, vorgesehen sein, um für eine besonders weiche und hautfreundliche Ausgestaltung des Fixierbandes zu sorgen. Polyurethan ist als Schaumstoff besonders gut geeignet.

In einer Variante ist auf der Innenseite des Fixierbandes (also auf derjenigen Seite, die in Kontakt mit der Haut eines Patienten kommt) eine Gummierung aufgebracht, für einen noch sicheren Halt auf der Haut bzw. den Haaren des Patienten zu sorgen.

In einer Variante besteht das Set nur aus den vorgenannten Elementen, gegebenenfalls unter Einbeziehung der vorstehend erläuterten alternativen Ausgestaltungsmöglichkeiten.

In einer Variante weisen die einzelnen Elemente des Sets zur Fixierung eines Katheterschlauchs lediglich Funktionsbereiche zur Ausbildung eines Klettverschlusses oder einer Klebeverbindung auf, nicht jedoch Funktionsbereiche zur Ausbildung einer anderen Verbindung. Dies vereinfacht den Herstellungsprozess und erleichtert die Anwendung des beanspruchten Sets.

Die Erfindung betrifft ferner die Verwendung des vorstehend erläuterten Sets zur Fixierung eines Katheterschlauchs zur Fixierung eines bei der extrakorporalen Membranoxygenierung eingesetzten doppellumigen Katheterschlauchs (ECMO-Katheterschlauchs), insbesondere in einem Kopfbereich oder einem Beinbereich eines Patienten.

Die Erfindung betrifft auch ein Verfahren zur Fixierung eines doppellumigen Katheterschlauchs an einem Patienten unter Verwendung eines Sets gemäß den vorherigen Erläuterungen, wobei das Verfahren die folgenden Schritte aufweist:
- Anbringen eines ersten Pflasters auf der Haut eines Patienten, wobei das erste Pflaster eine erste Seite aufweist, die einen Klebstoff aufweist, mittels dessen das erste Pflaster auf der Haut des Patienten angebracht wird, und das eine der ersten Seite gegenüberliegende zweite Seite aufweist, die einen Klettfunktionsbereich aufweist,
- Anbringen eines zweiten Pflasters auf der Haut des Patienten, wobei das zweite Pflaster eine erste Seite aufweist, die einen Klebstoff aufweist, mittels dessen das zweite Pflaster auf der Haut des Patienten angebracht wird, und das eine der ersten Seite gegenüberliegende zweite Seite aufweist, die einen Klettfunktionsbereich aufweist,
- Befestigen eines ersten Fixierelements an zwei Lumina des zu fixierenden doppellumigen Katheterschlauchs, indem das erste Fixierelement eine durch einen Klettverschluss verschlossene Schlaufe um die beiden Lumina bildet, wobei die gesamte nach außen weisende Oberfläche des ersten Fixierelements als Klettfunktionsbereich ausgestaltet ist, um ein besonders einfaches und positionsunabhängiges Fixieren des ersten Fixierelements an dem ersten Pflaster zu ermöglichen,
- Befestigen des ersten Fixierelements mit den zwei Lumina des zu fixierenden doppellumigen Katheterschlauchs an dem ersten Pflaster durch einen Klettverschluss zwischen dem Klettfunktionsbereich des ersten Fixierelements und dem Klettfunktionsbereich des ersten Pflasters,
- Herumlegen eines Fixierbandes, welches einen Klettfunktionsbereich, ein zweites Fixierelement und ein drittes Fixierelement, das unmittelbar benachbart zu dem zweiten Fixierelement angeordnet ist, aufweist, um einen Körperteil des Patienten,
- Befestigen des Fixierbandes an dem zweiten Pflaster durch einen Klettverschluss, der durch den Klettfunktionsbereich des Fixierbandes und den Klettfunktionsbereich des zweiten Pflasters ausgebildet wird,
- sofern das Fixierband in einem geöffneten Zustand vorliegt, in dem es zwar um den Körperteil des Patienten herumgelegt wurde, aber sich noch von alleine öffnen kann: Überführen des Fixierbandes von dem geöffneten Zustand in einen geschlossenen Zustand, in dem es sich, beispielsweise unter Ausbildung eines Klettverschlusses, nicht mehr von alleine öffnen kann,
- Befestigen eines ersten Lumens des zu fixierenden doppellumigen Katheterschlauchs an dem zweiten Fixierelement und
- Befestigen eines zweiten Lumens des zu fixierenden doppellumigen Katheterschlauchs an dem dritten Fixierelement, wobei das erste Lumen und das zweite Lumen getrennt durch das zweite Fixierelement und das dritte Fixierelement geführt werden.

Die vorgenannten Verfahrensschritte müssen nicht in der angegebenen Reihenfolge durchgeführt werden, sondern können auch in einer anderen, technisch sinnvollen Reihenfolge durchgeführt werden.

Ein solches Verfahren dient nicht im eigentlichen Sinne einer therapeutischen Behandlung des betreffenden Patienten, sondern unterstützt lediglich eine therapeutische Behandlung, indem ein im Rahmen der therapeutischen Behandlung eingesetzter Katheterschlauch in einer korrekten Position gehalten wird. Hierfür ist kein besonderer Eingriff in den Körper des Patienten erforderlich, da die einzelnen Elemente des beanspruchten Sets lediglich auf die Haut des Patienten aufgeklebt oder aufgelegt werden. Bestimmte Elemente des beanspruchten Sets, wie etwa das erste Fixierelement und das zweite Fixierelement, treten mit der Haut des Patienten gar nicht in Wechselwirkung, sondern werden von anderen Bestandteilen des Sets gehalten.

Das erste Pflaster wird in einer Variante auf eine Wange des Patienten geklebt. Das zweite Pflaster wird in einer Variante auf die Stirn eines Patienten geklebt.

In einer Variante ist das Fixierelement als Polstersteg ausgestaltet. In dieser Variante wird der zu fixierende Katheterschlauch in eine Klettschlaufe, die auf bzw. an dem Polstersteg ausgebildet ist, gelegt. Die Klettschlaufe wird dann geschlossen. Anschließend wird der Polstersteg mittels eines Klettverbinders mit dem Kopfband verbunden. Nachfolgend kann der Polstersteg dann mit dem ersten Pflaster verbunden werden.

Das Fixierband kann beispielsweise als flächig erstrecktes Band ausgestaltet sein.

Das Befestigen des einen Lumens des zu fixierenden doppellumigen Katheterschlauchs an dem zweiten Fixierelement erfolgt insbesondere dadurch, dass das zweite Fixierelement um das Lumen des zu fixierenden doppellumigen Katheterschlauchs herum gelegt wird und dabei eine durch einen Klettverschluss verschlossene Schlaufe bildet.

In einer Variante wird das Verfahren zur Fixierung eines ECMO-Katheterschlauchs verwendet, insbesondere im Kopfbereich eines Patienten.

Die Ausführungsbeispiele und Varianten, die weiter oben im Hinblick auf das beanspruchte Set zur Fixierung eines Katheterschlauchs erläutert wurden, sind in analoger Weise auf das beschriebene Verfahren übertragbar und anwendbar. Dabei können die einzelnen Varianten in jeder beliebigen Weise miteinander kombiniert wird.

Einzelne Aspekte der vorliegenden Erfindung werden nachfolgend anhand von Ausführungsbeispielen und Figuren näher erläutert. Es zeigen:
- Figur 1: eine schematische Ansicht eines Ausführungsbeispiels eines Sets zur Fixierung eines Katheterschlauchs;
- Figur 2: eine schematische Ansicht eines Ausführungsbeispiels eines Fixierbandes;
- Figur 3: eine Detaildarstellung eines Endbereichs des Fixierbandes der Figur 2;
- Figur 4: eine erste Detaildarstellung des Fixierbandes der Figur 2 im Bereich zweier Fixierelemente;
- Figur 5: eine zweite Detaildarstellung des Fixierbandes der Figur 2 im Bereich zweier Fixierelemente;
- Figur 6: eine erste Detaildarstellung des Fixierbandes der Figur 2 im Bereich eines Fixierelements, das einen zusätzlichen Haftbereich aufweist;
- Figur 7: eine zweite Detaildarstellung des Fixierbandes der Figur 2 im Bereich eines Fixierelements, das einen zusätzlichen Haftbereich aufweist;
- Figur 8: eine schematische Darstellung eines Ausführungsbeispiels eines ersten Pflasters;
- Figur 9: eine schematische Darstellung eines Ausführungsbeispiels eines ersten Fixierelements von seiner Außenseite;
- Figur 10: eine schematische Darstellung des Ausführungsbeispiels des ersten Fixierelements der Figur 9 von seiner Innenseite;
- Figur 11: eine schematische Darstellung eines Ausführungsbeispiels des Sets zur Fixierung eines Katheters bei bestimmungsgemäßer Benutzung als Set zur Fixierung eines ECMO-Katheterschlauchs;
- Figur 12: eine erste Darstellung eines weiteren Ausführungsbeispiels eines ersten Fixierelements;
- Figur 13: eine zweite Darstellung des Fixierelements der Figur 12;
- Figur 14: eine Detaildarstellung der Verbindung zwischen dem Fixierelement der Figur 12 und einem Fixierband; und
- Figur 15: eine Darstellung eines weiteren Ausführungsbeispiels eines Sets zur Fixierung eines Katheters unter Verwendung des Fixierelements der Figur 12 bei bestimmungsgemäßer Benutzung als Set zur Fixierung eines ECMO-Katheterschlauchs.

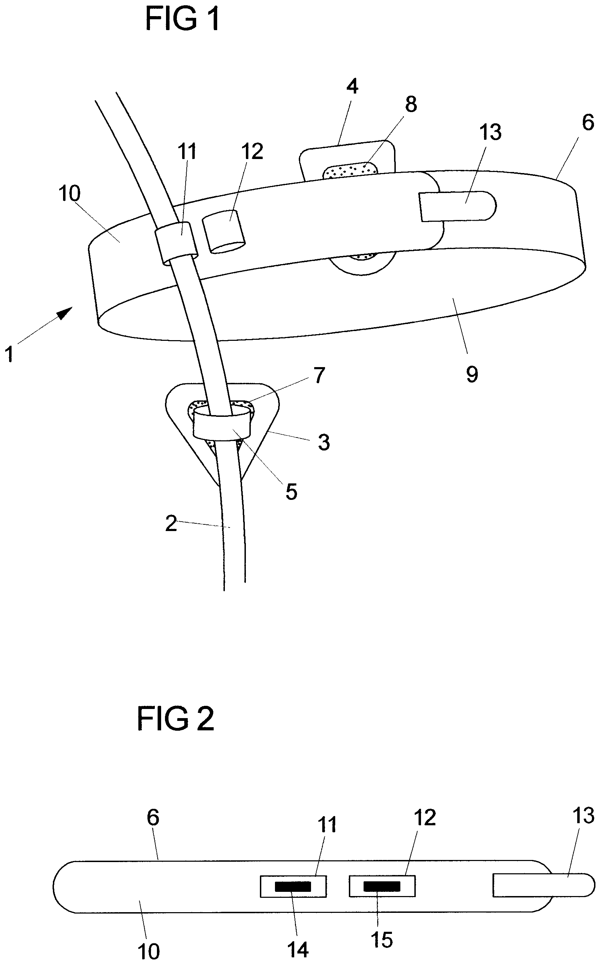

Die Figur 1 zeigt eine schematische Darstellung eines Sets 1, das zur Fixierung eines Katheterschlauchs 2 geeignet ist. Dieses Set 1 besteht aus einem Wangenpflaster 3, das als erstes Pflaster dient, einem identisch aufgebauten Stirnpflaster 4, das als zweites Pflaster dient, einer ersten Fixierschlaufe 5, die als erstes Fixierelement dient, und einem Fixierband 6. Auf einer in der Figur 1 nicht dargestellten Rückseite des Wangenpflasters 3 und auf einer in der Figur 1 ebenfalls nicht dargestellten Rückseite des Stirnpflasters 4 ist eine Klebeschicht angebracht, damit das Wangenpflaster 3 und das Stirnpflaster 4 auf den Wangenbereich bzw. Stirnbereich eines Patienten geklebt werden können.

Auf der Vorderseite des Wangenpflasters 3 ist ein Klettbereich 7 aus hartem Klettmaterial (z.B. Haken- oder Pilzkopfband) angeordnet, der als Klettfunktionsbereich dient. In gleicher Weise ist auf der Vorderseite des Stirnpflasters 4 ein Klettbereich 8, der aus hartem Klettmaterial besteht und als Klettfunktionsbereich dient, ausgebildet. Über den Klettbereich 7 des Wangenpflasters 3 ist die erste Fixierschlaufe 5 unter Ausbildung eines Klettverschlusses mit dem Wangenpflaster 3 verbunden. In einem Innenbereich der ersten Fixierschlaufe 5 ist der zu fixierende Katheterschlauch 2 aufgenommen. Da die erste Fixierschlaufe 5 den Katheterschlauch 2 fest umgibt und zusätzlich durch einen Haftbereich fixiert, wird auf diese Art und Weise eine rutschfeste Verbindung zwischen dem zu fixierenden Katheterschlauch 2 auf der einen Seite und dem Wangenpflaster 3 auf der anderen Seite realisiert.

Eine Innenseite 9 des Fixierbandes 6 weist eine veloursartige Oberfläche auf, die mit dem Klettbereich 8 des Stirnpflasters 4 einen Klettverschluss ausbildet und damit das Fixierband 6 an dem Stirnpflaster 4 festlegt. Auf einer der Innenseite 9 gegenüberliegenden Außenseite 10 des Fixierbandes 6 sind eine zweite Fixierschlaufe 11 als zweites Fixierelement und eine dritte Fixierschlaufe 12 als drittes Fixierelement angeordnet. Dabei sind die zweite Fixierschlaufe 11 und die dritte Fixierschlaufe 12 mit dem Fixierband 6 vernäht. Sie sind in der Darstellung der Figur 1 im geschlossenen Zustand gezeigt. In ihrem geöffneten Zustand stellen sich die zweite Fixierschlaufe 11 und die dritte Fixierschlaufe 12 als flächig erstreckte Bänder dar, die in ihrem mittleren Abschnitt mit dem Fixierband 6 vernäht sind.

Die zweite Fixierschlaufe 11 ist eng um den zu fixierenden Katheterschlauch 2 gelegt. Da sie in ihrem Innenbereich einen Klebstoffabschnitt aufweist, sorgt sie für einen guten Halt des zu fixierenden Katheterschlauchs 2 an sich selbst und damit an dem Fixierband 6. Da das Fixierband 6 seinerseits mittels des Stirnpflasters 4 am Kopf eines Patienten festgelegt werden kann, ergibt sich so eine feste Verbindung des zu fixierenden Katheterschlauchs 2 mit dem entsprechenden Patienten.

Die dritte Fixierschlaufe 12 dient zur Aufnahme eines weiteren Lumens eines Katheterschlauchs bzw. zur Aufnahme eines separaten Katheterschlauchs. Die erste Fixierschlaufe 5 ist derart dimensioniert, dass sie auch einen zweiten Katheterschlauch aufnehmen könnte.

Damit das Fixierband 6 gut um den Kopf (oder einen anderen Körperteil des betreffenden Patienten, sofern dies gewünscht ist) herumgelegt und verschlossen werden kann, ist auf der Außenseite 10 eine Klettlasche 13 ausgebildet, die mit der Oberfläche der Außenseite 10 einen Klettverschluss ausbildet. Statt einer separaten Klettlasche 13 könnte ein entsprechender Klettverschluss auch über einen in einem Endbereich des Fixierbandes 6 auf der Innenseite 9 vorgesehenen Klettbereich mit der Oberfläche der Außenseite 10 des Fixierbandes 6 ausgebildet werden.

Im Auslieferungszustand des Sets 1 sind die einzelnen Elemente des Sets 1 nicht in der in der Figur 1 dargestellten Art und Weise miteinander verbunden, sondern liegen als einzelne Elemente vor. Die in der Figur 1 gezeigte Darstellung gibt die Anordnung der einzelnen Elemente im konkreten Anwendungsfall des Sets 1 zur Fixierung eines ECMO-Katheters im Kopfbereich eines Patienten wieder.

Die Figur 2 zeigt eine Detaildarstellung des Fixierbandes 6 der Figur 1, wobei in der Figur 2 wie auch in allen folgenden Figuren stets dieselben Bezugszeichen für dieselben Elemente verwendet werden.

Das Fixierband 6 ist in der Figur 2 dabei von seiner Außenseite 10 in seinem geöffneten Zustand gezeigt. Das heißt, die Klettlasche 13 steht einseitig nicht im Eingriff mit der Oberfläche 10 des Fixierbandes 6, sodass das Band nicht geschlossen ist.

Auf der Außenseite 10 sind die bereits aus der Figur 1 bekannte zweite Fixierschlaufe 11 und dritte Fixierschlaufe 12 zu sehen. Diese sind in der Darstellung der Figur 2 ebenfalls in ihrem geöffneten Zustand gezeigt, sodass nun eine erste Klebefläche 14 als Haftbereich in einem mittigen Bereich der zweiten Fixierschlaufe 11 und eine zweite Klebefläche 15 als Haftbereich in einem mittigen Bereich der dritten Fixierschlaufe 12 zu sehen sind. Diese Klebeflächen 14, 15 dienen zur besonders guten Festlegung eines zu fixierenden Katheterschlauchs.

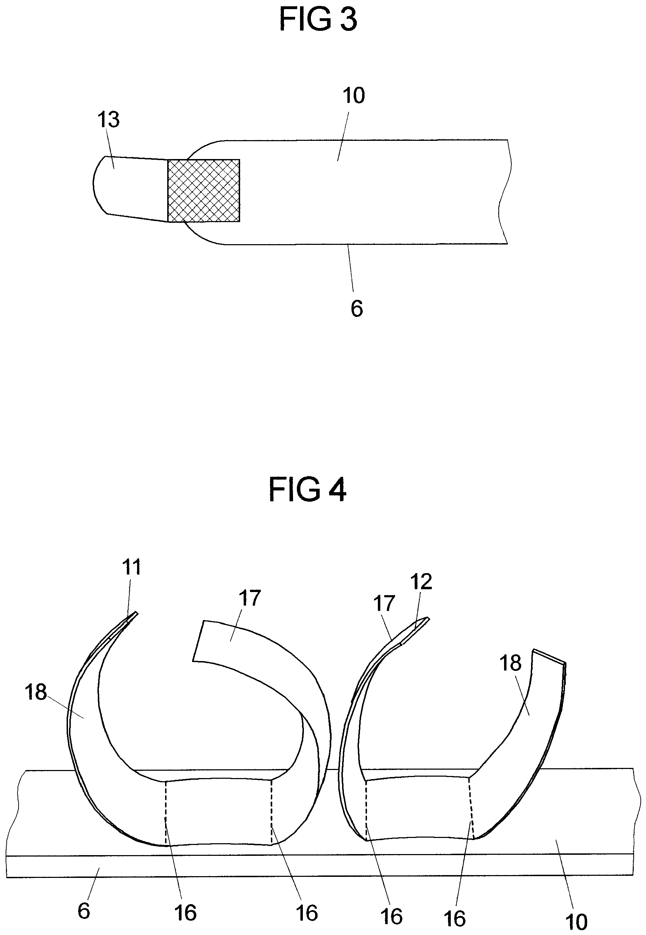

Die Figur 3 zeigt eine weitere Detaildarstellung des Fixierbandes 6 im Bereich der Klettlasche 13. In der Figur 3 ist die Verbindung zwischen der Klettlasche 13 und der Außenseite 10 des Fixierbandes 6 durch eine Verschweißung zu sehen.

Die Figur 4 zeigt eine Detaildarstellung des Fixierbandes 6 im Bereich der zweiten Fixierschlaufe 11 und der dritten Fixierschlaufe 12, die jeweils auch als Klettschlaufen bezeichnet werden können. Wie bereits oben erwähnt, sind die zweite Fixierschlaufe 11 und die dritte Fixierschlaufe 12 auf die Außenseite 10 des Fixierbandes 6 aufgenäht, und zwar jeweils mittels zweier Nähte 16. Die zweite Fixierschlaufe 11 und die dritte Fixierschlaufe 12 sind identisch aufgebaut. Sie bestehen aus einem weichen Klettbereich 17 auf ihrer Außenseite und einem harten Klettbereich 18 auf ihrer Innenseite. Auf diese Art und Weise können die zweite Fixierschlaufe 11 und die dritte Fixierschlaufe 12 um einen zu fixierenden Katheterschlauch zusammengerollt werden und sich selbst in der zusammengerollten Position fixieren, indem sie mit dem weichen Klettbereich 17 und dem harten Klettbereich 18 einen Klettverschluss ausbilden.

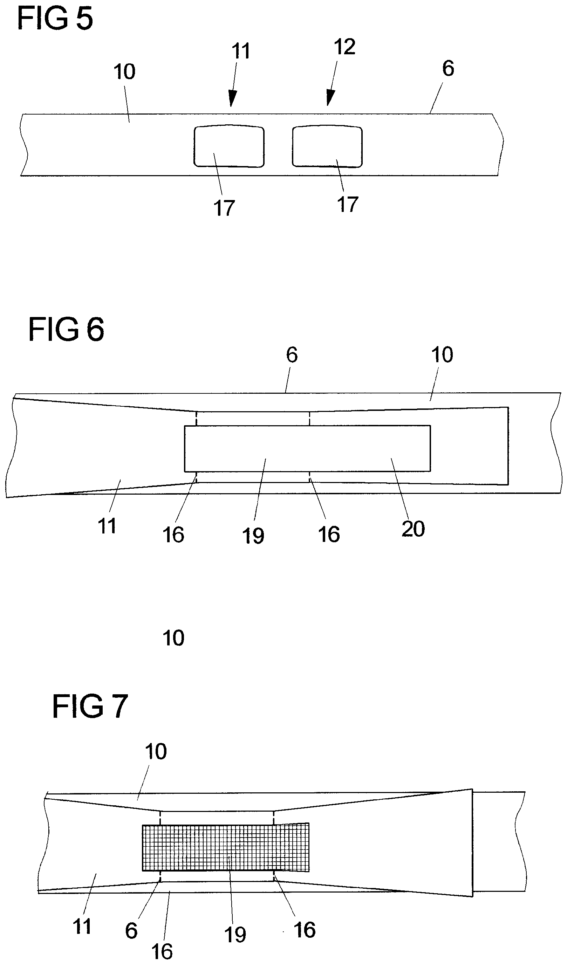

Dieser zusammengerollte Zustand der zweiten Fixierschlaufe 11 unter dritten Fixierschlaufe 12 ist in der Figur 5 dargestellt. In dieser Darstellung ist auch zu sehen, dass im zusammengerollten Zustand auf der Außenseite der zweiten Fixierschlaufe 11 und der dritten Fixierschlaufe 12 lediglich der weiche Klettbereich 17 positioniert ist.

Die Figur 6 zeigt abermals eine Darstellung der zweiten Fixierschlaufe 11 in ihrem geöffneten Zustand, wobei die in der Figur 6 dargestellte Fixierschlaufe 11 zusätzlich mit einer Klebefläche 19 versehen ist, die in der Darstellung der Figur 6 mit einer Abdeckfolie 20 überdeckt ist.

In der Figur 7 ist diese Abdeckfolie 20 nun entfernt, sodass die Klebefläche 19, die aus einem mit Klebstoff versehenen netzartigen Material besteht, gut sichtbar wird. Diese Klebefläche 19 sorgt - wie bereits oben erwähnt - für eine gute Fixierung des zu fixierenden Katheterschlauchs an der zweiten Fixierschlaufe 11 und damit an dem Fixierband 6.

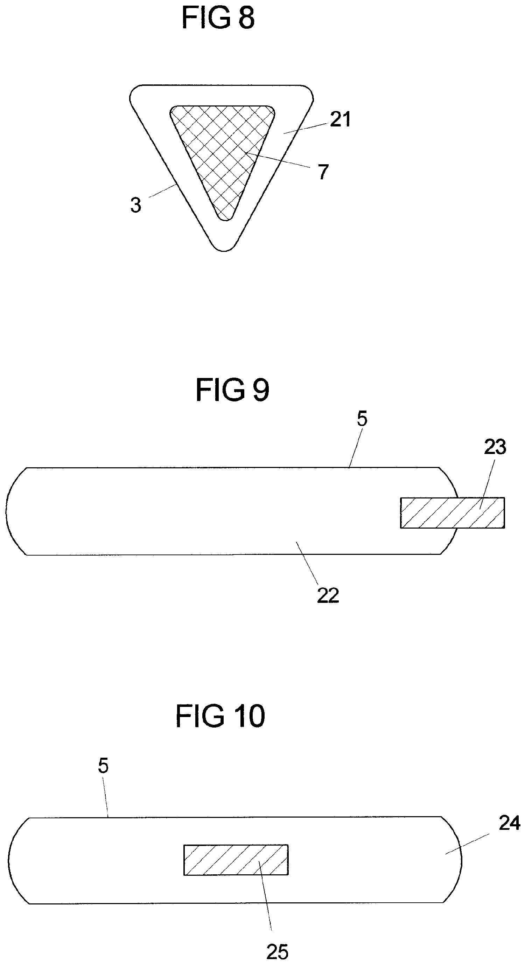

Die Figur 8 zeigt eine Detaildarstellung des Wangenpflasters 3. In einem mittigen Abschnitt des Wangenpflasters 3 ist der harte Klettbereich 7 angeordnet. Um Hautirritationen bei den betreffenden Patienten zu vermeiden, ist um den Klettbereich 7 ein umlaufender Rand 21 ausgebildet, sodass der Klettbereich 7 nicht in Kontakt mit der Haut des Patienten treten kann.

Wie bereits oben erwähnt, ist das Stirnpflaster 4 baugleich zum Wangenpflaster 3 aufgebaut, sodass die vorstehenden Erläuterungen in Bezug auf die Figur 8 auch auf das Stirnpflaster 4 übertragbar sind.

Die Figur 9 zeigt eine Detaildarstellung der ersten Fixierschlaufe 5, die eine veloursartige Oberfläche 22 aufweist, an der an einem Ende eine Klettlasche 23 angebracht ist. Damit wird die Verbindung zwischen der Klettlasche 23 und der Oberfläche 22 der ersten Fixierschlaufe 5 in gleicher Weise realisiert wie die Verbindung zwischen der Klettlasche 13 und der Oberfläche 10 des Fixierbandes 6.

Die Figur 10 zeigt eine schematische Darstellung einer Innenseite 24 der ersten Fixierschlaufe 5. Auf dieser Innenseite 24 ist abermals eine Klebefläche 25 angeordnet, die von ihrem Aufbau her den bereits in Zusammenhang mit den Figuren 2, 6 und 7 erläuterten Klebeflächen des Fixierbandes 6 und der zweiten Fixierschlaufe 11 entspricht.

In einer alternativen Ausgestaltung der ersten Fixierschlaufe 5 weist diese keine Klettlasche 23 auf. Vielmehr ist dann die Innenseite 24 zumindest abschnittsweise als harter Klettfunktionsbereich ausgestaltet, sodass die erste Fixierschlaufe 5 - ähnlich wie die zweite Fixierschlaufe 11 und die dritte Fixierschlaufe 12 - zusammengerollt werden kann, sodass ein Klettverschluss zwischen der Außenseite 22 und der Innenseite 24 ausgebildet wird.

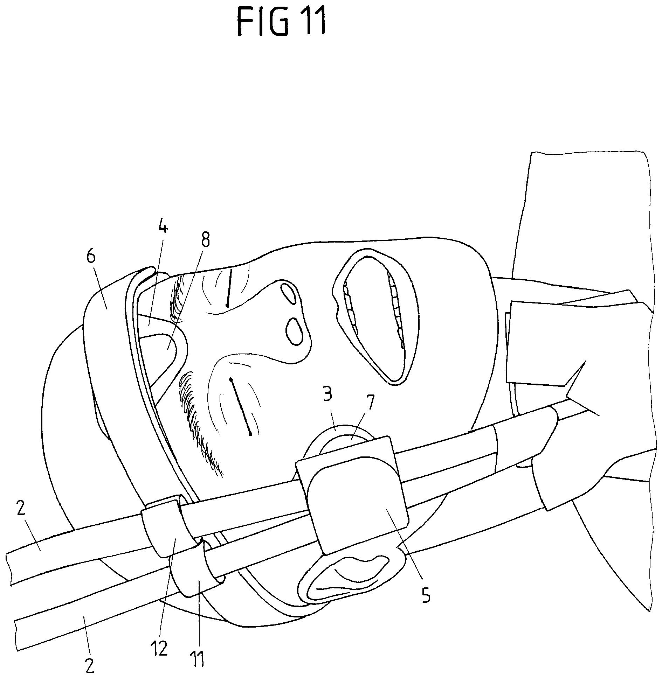

Die Figur 11 zeigt eine schematische Darstellung des Sets 1 im konkreten Anwendungsbereich der Fixierung eines ECMO-Katheters am Kopf eines Patienten. Die Anbringung des Sets zur Katheterfixierung wird nachfolgend nochmals im Detail beschrieben.

Zunächst wird das Wangenpflaster 3 auf eine Wange des Patienten geklebt, und zwar auf der Seite des Patienten, an der die Einstichstelle des betreffenden ECMO-Katheters liegt. Anschließend wird das Stirnpflaster 4 auf die Stirn des Patienten geklebt, und zwar in einem mittigen Bereich (in etwa oberhalb der Nase).

Nun wird das Fixierband 6 um den Kopf des Patienten gelegt, sodass die zweite Fixierschlaufe 11 und die dritte Fixierschlaufe 12 auf einer gedachten Verbindungslinie zum Wangenpflaster 3 möglichst gerade oberhalb des Wangenpflasters 3 angeordnet sind. Nun wird das Fixierband 6 auf das Stirnpflaster 4 aufgedrückt, sodass eine feste Verbindung zwischen dem Stirnpflaster 4 und dem Fixierband 6 hergestellt wird. Das Fixierband 6 wird dann mittels des integrierten Klettverschlusses geschlossen. Der entsprechende Klettverschluss ist in der Figur 11 nicht zu sehen.

Nun wird die erste Fixierschlaufe 5 in etwa in Höhe des Wangenpflasters 3 um den Katheterschlauch 2 gelegt und mithilfe eines durch die erste Fixierschlaufe 5 selbst ausgebildeten Klettverschlusses verschlossen. Dabei bietet die auf der Innenseite der ersten Fixierschlaufe 5 angeordnete Klebefläche, die in der Figur 11 nicht zu sehen ist, dem zu fixierenden Katheterschlauch 2 zusätzlichen Halt. Die erste Fixierschlaufe 5 mit dem innenliegenden Katheter 2 wird nun auf das Wangenpflaster 3 gedrückt, sodass ein Klettverschluss zwischen der ersten Fixierschlaufe 5 und dem Wangenpflaster 3 ausgebildet wird, der den zu fixierenden Katheter am Wangenpflaster 3 fixiert.

Nun werden die zweite Fixierschlaufe 11 und die dritte Fixierschlaufe 12 um den zu fixierenden Katheterschlauch 2 herumgelegt und ebenfalls mittels des durch die Fixierschlaufen 11, 12 selbst bereitgestellten Klettverschlusses verschlossen. Dabei sorgen abermals die auf den Innenseiten der zweiten Fixierschlaufe 11 unter dritten Fixierschlaufe 12 angeordneten Klebeflächen für einen zusätzlichen Halt des Katheterschlauchs 2.

Der fixierte ECMO-Katheter ist nun im Bereich der Einstichstelle an der Vena jugularis zugentlastet und vor einem unbeabsichtigten Dislozieren geschützt.

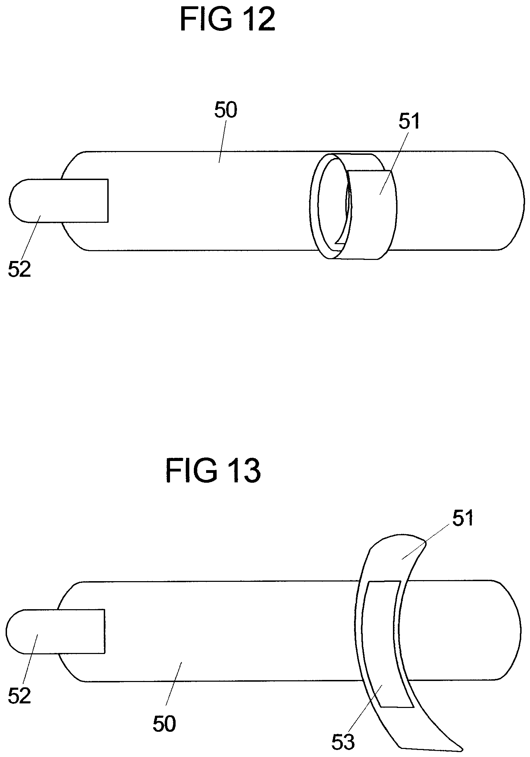

Die Figur 12 zeigt einen Polstersteg 50, der in einer Variante als erstes Fixierelement eingesetzt werden kann. Dieser Polstersteg 50 besteht aus einem Schaumstoff, der beidseitig veloursbeschichtet ist. Der Polstersteg 50 ist ca. 15 cm lang. Er weist beidseits abgerundete Ecken auf, um für einen besonders hohen Tragekomfort zu sorgen.

Im unteren Drittel des Polsterstegs 50 (in der Darstellung der Figur 12 rechts dargestellt) ist eine Klettschlaufe 51 auf dem Polstersteg 50 aufgenäht. Diese Klettschlaufe 51 ist ähnlich wie die zweite Fixierschlaufe 11 und die dritte Fixierschlaufe 12 ausgestaltet. Die Größe der Klettschlaufe 51 ist dabei derart gewählt, dass sowohl ein einlumigen Katheterschlauch als auch ein doppellumigen Katheterschlauch durch die Klettschlaufe 51 fixiert werden kann.

Im oberen Bereich des Polsterstegs 50 (in der Darstellung der Figur 12 links dargestellt) ist ein Klettverbinder 52 vorgesehen, der auf dem Polstersteg 50 aufgenäht oder aufgeschweißt ist. Der Klettverbinder 52 dient dazu, den Polstersteg 50 mit dem Fixierband 6 zu verbinden. Dabei kann der Klettverbinder 52 beispielsweise zwischen der zweiten Fixierschlaufe 11 und der dritten Fixierschlaufe 12 angeordnet werden (vergleiche hierzu Figur 1).

Die Figur 13 zeigt den Polstersteg 50 mit geöffneter Klettschlaufe 51. In dieser Darstellung ist zu erkennen, dass in einem mittleren Bereich der Klettschlaufe 51 eine Klebefläche 53 angeordnet ist. Diese Klebefläche 53 dient zur besseren Fixierung des durch die Klettschlaufe 51 durchgeführten Katheterschlauchs. Insoweit wird auf die Erläuterungen zu den Klebeflächen 14, 15 der zweiten Fixierschlaufe 11 unter dritten Fixierschlaufe 12 verwiesen (vergleiche Figur 2 und die diesbezüglichen Erläuterungen).

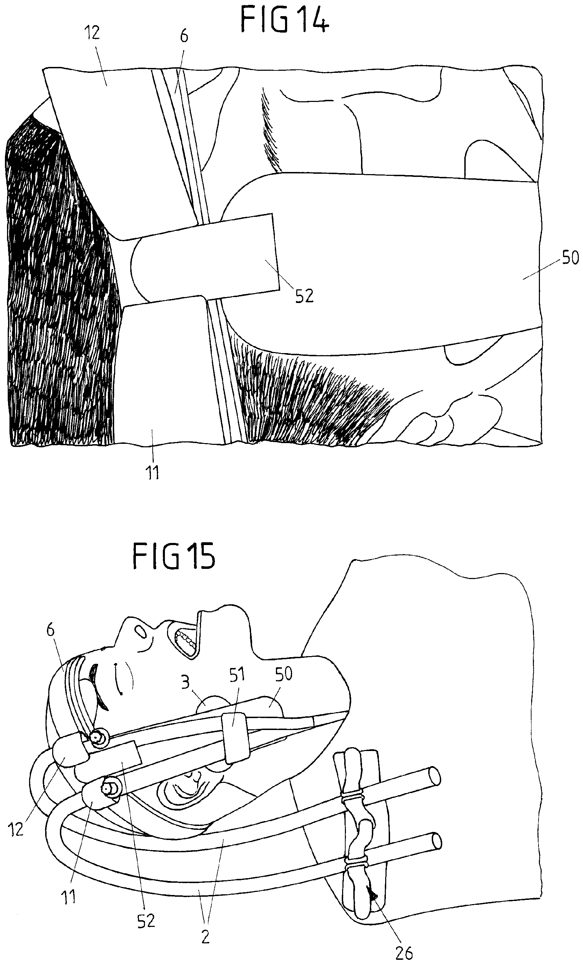

In der Figur 14 ist dargestellt, wie der Polstersteg 50 mit dem bereits aus der Figur 1 bekannten Fixierband 6 verbunden werden kann. Wie bereits oben erwähnt, wird zu diesem Zweck der Klettverbinder 52 zwischen der zweiten Fixierschlaufe 11 und der dritten Fixierschlaufe 12 positioniert. Dadurch wird dann der Polstersteg 50 fest am Fixierband 6 gehalten.

Die Figur 15 zeigt den Einsatz des Polsterstegs 50 bei bestimmungsgemäßer Verwendung als Fixierelement im Rahmen einer ECMO-Katheterfixierung. Bei der Beschreibung der Figur 15 wird auf die Beschreibung der Figur 11 Bezug genommen. Der Polstersteg 50 ist auf das Wangenpflaster 3 aufgelegt und wird durch dieses fixiert. Ein doppellumiger Katheterschlauch 2 ist durch die Klettschlaufe 51 geführt und wird durch diese am Polstersteg 50 fixiert. Der Polstersteg 50 ist - wie bereits in der Figur 14 gezeigt - durch den Klettverbinder 52 mit dem Fixierband 6 verbunden.

Der doppellumige Katheterschlauch 2 wird durch die zweite Fixierschlaufe 11 und die dritte Fixierschlaufe 12 am Fixierband 6 gehalten. Die Verbindung zwischen dem Fixierband 6 und dem Wangenpflaster 3 wird in dieser Ausführungsvariante nicht nur über den Katheterschlauch 2, sondern auch über den Polstersteg 50 realisiert.

Der Katheterschlauch 2 ist im Schulterbereich eines Patienten zudem durch eine herkömmliche Katheterfixierung 26 festgelegt. Hierfür kann beispielsweise das Produkt Main-Lock der Anmelderin verwendet werden, das als zusätzlicher Bestandteil des vorliegend beanspruchten Sets zur Fixierung eines Katheterschlauchs angeboten werden kann.

Der Polstersteg 50 wird üblicherweise derart eingesetzt, dass zunächst das Wangenpflaster 3 auf der Haut des Patienten im Wangenbereich aufgeklebt wird. Anschließend wird der Polstersteg 50 mithilfe des Klettverbinders 52 an dem bereits zuvor angelegten Fixierband 6 befestigt. Dann wird der Polstersteg 50 auf den Klettfunktionsbereich des Wangenpflasters 3 aufgelegt und unter Ausbildung einer Klettverbindung mit dem Wagenpflaster 3 verbunden. Nun wird der zu fixierende Katheterschlauch 2 in die auf dem Polstersteg 50 befindliche Klettschlaufe 51 eingelegt und mit der dort vorhandenen Klebefläche 53 festgelegt. Die Klettschlaufe 51 wird dann geschlossen. Im nächsten Schritt wird der Katheter in die zweite Fixierschlaufe 11 und die dritte Fixierschlaufe 12 des Fixierbandes 6 eingelegt. Die zweite Fixierschlaufe 11 und die dritte Fixierschlaufe 12 werden dann geschlossen. Durch die Klettschlaufe 51, die zweite Fixierschlaufe 11 und die dritte Fixierschlaufe 12 mit den zugehörigen Klebeflächen erfolgt eine effektive Fixierung des Katheterschlauchs 2.

Neben der Fixierung und Zugentlastung an der Einstichstelle des zu fixierenden Katheterschlauchs bietet der Polstersteg 50 aufgrund einer Materialbeschaffenheit eine Polsterung zwischen dem zu fixierenden Katheterschlauch und der Gesichtshaut des Patienten. Denn der Katheterschlauch 2 wird nicht direkt auf der Gesichtshaut des Patienten geführt, sondern auf dem Polstersteg 50. Das heißt, der Polstersteg 50 vermittelt eine Polsterwirkung bei der Fixierung des Katheterschlauchs 2.

Da der Polstersteg 50 mittels des Klettverbinders 52 einfach mit dem Fixierband 6 verbunden werden kann und auch wieder einfach vom Fixierband 6 entfernt werden kann, ist es möglich, unabhängig vom Fixierband 6 einen Wechsel des Polsterstegs 50 durchzuführen, falls dies erforderlich werden sollte.

Da der Polstersteg 50 sowohl mit der Wange des Patienten (nämlich über das Wangenpflaster 3) als auch mit dem Stirnbereich des Patienten (über das dort fixierte Fixierband 6) verbunden ist, bietet der Polstersteg 50 einen zusätzlichen Schutz vor dem Verrutschen der Fixierung des zu fixierenden Katheterschlauchs 2 in Längsrichtung des Katheterschlauchs 2.

## Patentansprüche

1. Set zur Fixierung eines doppellumigen Katheterschlauchs, **gekennzeichnet durch**
• ein erstes Pflaster (3) mit einer ersten Seite, die einen Klebstoff aufweist, mittels dessen das erste Pflaster (3) auf der Haut eines Patienten angebracht werden kann, und mit einer der ersten Seite gegenüberliegenden zweiten Seite, die einen Klettfunktionsbereich (7) aufweist,
• ein zweites Pflaster (4) mit einer ersten Seite, die einen Klebstoff aufweist, mittels dessen das zweite Pflaster (4) auf der Haut des Patienten angebracht werden kann, und mit einer der ersten Seite gegenüberliegenden zweiten Seite, die einen Klettfunktionsbereich (8) aufweist,
• ein erstes Fixierelement (5), das zum Fixieren zweier Lumina eines doppellumigen Katheterschlauchs (2) an dem ersten Pflaster (3) dient, **gekennzeichnet dadurch, dass** das erste Fixierelement (5) eine durch einen Klettverschluss verschlossene Schlaufe um die beiden Lumina bildet, wobei die gesamte nach außen weisende Oberfläche des ersten Fixierelements als Klettfunktionsbereich ausgestaltet ist, um ein besonders einfaches und positionsunabhängiges Fixieren des ersten Fixierelements (5) an dem ersten Pflaster (3) zu ermöglichen, wobei der Klettfunktionsbereich des ersten Fixierelements (5) mit dem Klettfunktionsbereich (7) des ersten Pflasters (3) einen Klettverschluss ausbildet, und
• ein Fixierband (6), das einen Klettfunktionsbereich aufweist und das dazu vorgesehen ist, um einen Körperteil des Patienten herumgeführt und mittels des zweiten Pflasters (4) unter Ausbildung eines Klettverschlusses zwischen dem Klettfunktionsbereich des Fixierbandes (6) und dem Klettfunktionsbereich (8) des zweiten Pflasters (4) an dem Körperteil des Patienten fixiert zu werden, wobei das Fixierband (6) ein zweites Fixierelement (11) aufweist, das zum Fixieren eines ersten Lumens des doppellumigen Katheterschlauchs (2) an dem Fixierband (6) dient und wobei das Fixierband (6) ein drittes Fixierelement (12) aufweist, das unmittelbar benachbart zu dem zweiten Fixierelement (11) angeordnet ist und das zum Fixieren eines zweiten Lumens des doppellumigen Katheterschlauchs (2) an dem Fixierband (6) dient, wobei sich das erste Lumen und das zweite Lumen getrennt durch das zweite Fixierelement (11) und das dritte Fixierelement (12) führen lassen.

2. Set gemäß Anspruch 1, **dadurch gekennzeichnet, dass** das erste Pflaster (3) und das zweite Pflaster (4) baugleich sind.

3. Set gemäß Anspruch 1 oder 2, **dadurch gekennzeichnet, dass** das erste Fixierelement (5) und/oder das zweite Fixierelement (11) als flächig erstrecktes Band mit einer ersten Seite, die einen harten Klettfunktionsbereich (18) aufweist, und einer der ersten Seite gegenüberliegenden zweiten Seite, die einen weichen Klettfunktionsbereich (17) aufweist, ausgestaltet sind.

4. Set gemäß einem der vorherigen Ansprüche, **dadurch gekennzeichnet, dass** das zweite Fixierelement (11) fest mit dem Fixierband (6) verbunden ist.

5. Set gemäß einem der vorherigen Ansprüche, **dadurch gekennzeichnet, dass** das erste Fixierelement (5) und/oder das zweite Fixierelement (11) einen Haftbereich (14, 19, 25) aufweisen, der dazu dient, eine zusätzliche Haltekraft gegenüber einem zu fixierenden doppellumigen Katheterschlauch (2) aufzubauen, so dass eine rutschfeste Verbindung des zu fixierenden Katheterschlauchs mit dem ersten Fixierelement (5) und/oder dem zweiten Fixierelement (11) und damit dem gesamten Set ermöglicht wird.

6. Set nach Anspruch 5, **dadurch gekennzeichnet, dass** der Haftbereich durch ein mit einem Klebstoff versehenes netzartiges Element ausgebildet ist.

7. Set gemäß einem der vorherigen Ansprüche, **dadurch gekennzeichnet, dass** das Fixierband (6) an einem seiner Enden einen Klettfunktionsbereich (13) aufweist, der mit einem Oberflächenabschnitt des Fixierbandes (6) einen Klettverschluss bildet.

8. Set gemäß einem der vorherigen Ansprüche, **dadurch gekennzeichnet, dass** das Fixierband (6), abgesehen von dem zweiten Fixierelement (11) und dem dritten Fixierelement (12) sowie abgesehen von einem optionalen Klettfunktionsbereich (13) an einem seiner Enden, eine einheitlich ausgebildete Oberfläche einer ersten Seite (10) und/oder einer der ersten Seite gegenüberliegenden zweiten Seite (9) aufweist.

9. Verfahren zur Fixierung eines doppellumigen Katheterschlauchs an einem Patienten unter Verwendung eines Sets gemäß einem der Ansprüche 1 bis 8, **gekennzeichnet durch** die folgenden Schritte:
• Anbringen eines ersten Pflasters (3) auf der Haut eines Patienten, wobei das erste Pflaster (3) eine erste Seite aufweist, die einen Klebstoff aufweist, mittels dessen das erste Pflaster auf der Haut des Patienten angebracht wird, und das eine der ersten Seite gegenüberliegende zweite Seite aufweist, die einen Klettfunktionsbereich (7) aufweist,
• Anbringen eines zweiten Pflasters (4) auf der Haut des Patienten, wobei das zweite Pflaster eine erste Seite aufweist, die einen Klebstoff aufweist, mittels dessen das zweite Pflaster auf der Haut des Patienten angebracht wird, und das eine der ersten Seite gegenüberliegende zweite Seite aufweist, die einen Klettfunktionsbereich (8) aufweist,
• Herumlegen eines Fixierbandes (6), welches einen Klettfunktionsbereich, ein zweites Fixierelement (11) und ein drittes Fixierelement (12), das unmittelbar benachbart zu dem zweiten Fixierelement (11) angeordnet ist, aufweist, um einen Körperteil des Patienten,
• Befestigen des Fixierbandes (6) an dem zweiten Pflaster (4)durch einen Klettverschluss, der durch den Klettfunktionsbereich des Fixierbandes (6) und den Klettfunktionsbereich des zweiten Pflasters (4) ausgebildet wird,
• sofern das Fixierband (6) in einem geöffneten Zustand vorliegt, in dem es zwar um den Körperteil des Patienten herumgelegt wurde, aber sich noch von alleine öffnen kann: Überführen des Fixierbandes (6) von dem geöffneten Zustand in einen geschlossenen Zustand, in dem es sich, beispielsweise unter Ausbildung eines Klettverschlusses, nicht mehr von alleine öffnen kann,
• Befestigen eines ersten Fixierelements (5) an zwei Lumina des zu fixierenden doppellumigen Katheterschlauchs (2), indem das erste Fixierelement (5) eine durch einen Klettverschluss verschlossene Schlaufe um die beiden Lumina bildet, wobei die gesamte nach außen weisende Oberfläche des ersten Fixierelements (5) als Klettfunktionsbereich ausgestaltet ist, um ein besonders einfaches und positionsunabhängiges Fixieren des ersten Fixierelements (5) an dem ersten Pflaster (3) zu ermöglichen,
• Befestigen des ersten Fixierelements (5) mit den zwei Lumina des zu fixierenden doppellumigen Katheterschlauchs (2) an dem ersten Pflaster (3) durch einen Klettverschluss zwischen dem Klettfunktionsbereich des ersten Fixierelements (5) und dem Klettfunktionsbereich (7) des ersten Pflasters (3),
• Befestigen eines ersten Lumens des zu fixierenden doppellumigen Katheterschlauchs (2) an dem zweiten Fixierelement (11) und
• Befestigen eines zweiten Lumens des zu fixierenden doppellumigen Katheterschlauchs (2) an dem dritten Fixierelement (12), wobei das erste Lumen und das zweite Lumen getrennt durch das zweite Fixierelement (11) und das dritte Fixierelement (12) geführt werden.

## Claims

1. A kit for fixing a double-lumen catheter hose, **characterized by**
- a first plaster (3) with a first side, which has an adhesive by means of which the first plaster (3) can be applied to the skin of a patient, and with a second side opposite the first side, which has a hook-and-loop function area (7),
- a second plaster (4) with a first side, which has an adhesive by means of which the second plaster (4) can be applied to the skin of the patient, and with a second side opposite the first side, which has a hook-and-loop function area (8),
- a first fixing element (5) which serves for fixing two lumina of a double-lumen catheter hose (2) on the first plaster (3), **characterized in that** the first fixing element (5) forms a loop closed by a hook-and-loop fastener around the two lumina, wherein the entire outwardly facing surface of the first fixing element is designed as a hook-and-loop function area in order to provide for a particularly easy and position-independent fixation of the first fixing element (5) on the first plaster (3), wherein the hook-and-loop function area of the first fixing element (5) together with the hook-and-loop function area (7) of the first plaster (3) forms a hook-and-loop fastener, and
- a fixing tape (6) which has a hook-and-loop function area and is provided to be guided around a body part of the patient and to be fixed on the body part of the patient by means of the second plaster (4) by forming a hook-and-loop fastener between the hook-and-loop function area of the fixing tape (6) and the hook-and-loop function area (8) of the second plaster (4), wherein the fixing tape (6) has a second fixing element (11) which serves for fixing a first lumen of the double-lumen catheter hose (2) on the fixing tape (6), and wherein the fixing tape (6) has a third fixing element (12) which is arranged directly adjacent to the second fixing element (11) and which serves for fixing a second lumen of the double-lumen catheter hose (2) on the fixing tape (6), wherein the first lumen and the second lumen let themselves be separately guided through the second fixing element (11) and the third fixing element (12).

2. The kit according to claim 1, **characterized in that** the first plaster (3) and the second plaster (4) are identically constructed.

3. The kit according to claim 1 or 2, **characterized in that** the first fixing element (5) and/or the second fixing element (11) are designed as a flatly extended tape with a first side, which has a hard hook-and-loop function area (18), and a second side opposite the first side, which has a soft hook-and-loop function area (17).

4. The kit according to any of the preceding claims, **characterized in that** the second fixing element (11) is firmly connected with the fixing tape (6).

5. The kit according to any of the preceding claims, **characterized in that** the first fixing element (5) and/or the second fixing element (11) have an adhesive area (14, 19, 25) which serves to build up an additional holding force with respect to a double-lumen catheter hose (2) to be fixed, so that a non-slip connection of the catheter hose to be fixed with the first fixing element (5) and/or the second fixing element (11) and hence the entire kit becomes possible.

6. The kit according to claim 5, **characterized in that** the adhesive area is formed by a net-like element provided with an adhesive.

7. The kit according to any of the preceding claims, **characterized in that** the fixing tape (6) has a hook-and-loop function area (13) at one of its ends, which forms a hook-and-loop fastener with a surface portion of the fixing tape (6).

8. The kit according to any of the preceding claims, **characterized in that** the fixing tape (6), apart from the second fixing element (11) and the third fixing element (12) and apart from an optional hook-and-loop function area (13) at one of its ends, has a uniformly configured surface of a first side (10) and/or of a second side (9) opposite the first side.

9. A method for fixing a double-lumen catheter hose on a patient by using a kit according to any of claims 1 to 8, **characterized by** the following steps:
- applying a first plaster (3) to the skin of a patient, wherein the first plaster (3) has a first side, which has an adhesive by means of which the first plaster is applied to the skin of the patient, and which has a second side opposite the first side, which has a hook-and-loop function area (7),
- applying a second plaster (4) to the skin of the patient, wherein the second plaster has a first side, which has an adhesive by means of which the second plaster is applied to the skin of the patient, and which has a second side opposite the first side, which has a hook-and-loop function area (8),
- placing a fixing tape (6), which has a hook-and-loop function area, a second fixing element (11) and a third fixing element (12), which is arranged directly adjacent to the second fixing element (11), around a body part of the patient,
- fixing the fixing tape (6) on the second plaster (4) by a hook-and-loop fastener, which is formed by the hook-and-loop function area of the fixing tape (6) and the hook-and-loop function area of the second plaster (4),
- in case the fixing tape (6) is present in an open condition in which it has been placed around the body part of the patient, but can still open on its own: transferring the fixing tape (6) from the open condition into a closed condition in which it no longer can open on its own, for example by forming a hook-and-loop fastener,
- fixing a first fixing element (5) on two lumina of the double-lumen catheter hose (2) to be fixed, in that the first fixing element (5) forms a loop closed by a hook-and-loop fastener around the two lumina, wherein the entire outwardly facing surface of the first fixing element (5) is designed as a hook-and-loop function area in order to provide for a particularly easy and position-independent fixation of the first fixing element (5) on the first plaster (3),
- fixing the first fixing element (5) with the two lumina of the double-lumen catheter hose (2) to be fixed on the first plaster (3) by a hook-and-loop fastener between the hook-and-loop function area of the first fixing element (5) and the hook-and-loop function area (7) of the first plaster (3),
- fixing a first lumen of the double-lumen catheter hose (2) to be fixed on the second fixing element (11), and
- fixing a second lumen of the double-lumen catheter hose (2) to be fixed on the third fixing element (12), wherein the first lumen and the second lumen are separately guided through the second fixing element (11) and the third fixing element (12).

## Revendications

1. Ensemble pour la fixation d'un tube de cathéter à double lumière, **caractérisé par**
- un premier pansement (3) avec un premier côté qui présente un adhésif au moyen duquel le premier pansement (3) peut être appliqué sur la peau d'un patient, et avec un deuxième côté opposé au premier côté, qui présente une zone ayant une fonction de velcro (7),
- un deuxième pansement (4) avec un premier côté qui présente un adhésif au moyen duquel le deuxième pansement (4) peut être appliqué sur la peau du patient, et avec un deuxième côté opposé au premier côté, qui présente une zone ayant une fonction de velcro (8),
- un premier élément de fixation (5) qui sert à la fixation de deux lumières d'un tube de cathéter à double lumière (2) sur le premier pansement (3), **caractérisé en ce que** le premier élément de fixation (5) forme une boucle fermée par une fermeture velcro autour des deux lumières, dans lequel toute la surface tournée vers l'extérieur du premier élément de fixation est configurée comme une zone ayant une fonction de velcro, pour permettre une fixation particulièrement simple et indépendante de la position du premier élément de fixation (5) sur le premier pansement (3), dans lequel la zone ayant une fonction de velcro du premier élément de fixation (5) avec la zone ayant une fonction de velcro (7) du premier pansement (3) forme une fermeture velcro, et
- une bande de fixation (6) qui présente une zone ayant une fonction de velcro et qui est prévue pour être passée autour d'une partie du corps du patient et pour être fixée au moyen du deuxième pansement (4) en formant une fermeture velcro entre la zone ayant une fonction de velcro de la bande de fixation (6) et la zone ayant une fonction de velcro (8) du deuxième pansement (4) sur la partie du corps du patient, dans lequel la bande de fixation (6) présente un deuxième élément de fixation (11) qui sert à la fixation d'une première lumière du tube de cathéter à double lumière (2) sur la bande de fixation (6) et dans lequel la bande de fixation (6) présente un troisième élément (12) qui est disposé immédiatement adjacent au deuxième élément de fixation (11) et qui sert à la fixation d'une deuxième lumière du tube de cathéter à double lumière (2) sur la bande de fixation (6), dans lequel la première lumière et la deuxième lumière sont conduites séparément au travers du deuxième élément de fixation (11) et du troisième élément de fixation (12).

2. Ensemble selon la revendication 1, **caractérisé en ce que** le premier pansement (3) et le deuxième pansement (4) sont de configuration identique.

3. Ensemble selon la revendication 1 ou 2, **caractérisé en ce que** le premier élément de fixation (5) et/ou le deuxième élément de fixation (11) sont configurés sous la forme d'une bande qui s'étend de façon plane, avec un premier côté qui présente une zone ayant une fonction de velcro dure (18) et un deuxième côté opposé au premier côté qui présente une zone ayant une fonction de velcro souple (17).

4. Ensemble selon l'une des revendications précédentes, **caractérisé en ce que** le deuxième élément de fixation (11) est relié fixement à la bande de fixation (6).

5. Ensemble selon l'une des revendications précédentes, **caractérisé en ce que** le premier élément de fixation (5) et/ou le deuxième élément de fixation (11) présentent une zone adhésive (14, 19, 25) qui sert à constituer une force adhésive supplémentaire par rapport à un tube de cathéter à double lumière à fixer (2), de sorte qu'une liaison anti-glissement du tube de cathéter à fixer avec le premier élément de fixation (5) et/ou le deuxième élément de fixation (11) et ainsi l'ensemble complet soit possible.

6. Ensemble selon la revendication 5, **caractérisé en ce que** la zone adhésive est conçue par un élément de type filet doté d'un adhésif.

7. Ensemble selon l'une des revendications précédentes, **caractérisé en ce que** la bande de fixation (6) présente à l'une de ses extrémités, une zone ayant une fonction de velcro (13) qui forme avec un segment de surface de la bande de fixation (6), une fermeture adhésive.

8. Ensemble selon l'une des revendications précédentes, **caractérisé en ce que** la bande de fixation (6), en dehors du deuxième élément de fixation (11) et du troisième élément de fixation (12) et également en dehors d'une zone ayant une fonction de velcro facultative (13) sur l'une de ses extrémités, présente une surface formée de façon unitaire d'un premier côté (10) et/ou d'un deuxième côté opposé au premier côté (9).

9. Procédé de fixation d'un tube de cathéter à double lumière sur un patient en utilisant un ensemble selon l'une des revendications 1 à 8, **caractérisé par** les étapes suivantes :
- application d'un premier pansement (3) sur la peau d'un patient, dans lequel le premier pansement (3) présente un premier côté qui présente un adhésif au moyen duquel le premier pansement est appliqué sur la peau du patient, et qui présente un deuxième côté opposé au premier côté, qui présente une zone ayant une fonction de velcro (7),
- application d'un premier pansement (4) sur la peau du patient, dans lequel le deuxième pansement présente un premier côté qui présente un adhésif au moyen duquel le deuxième pansement est appliqué sur la peau du patient, et qui présente un deuxième côté opposé au premier côté, qui présente une zone ayant une fonction de velcro (8),
- placement d'une bande de fixation (6) qui présente une zone ayant une fonction de velcro, un deuxième élément de fixation (11) et un troisième élément de fixation (12), qui est disposé immédiatement adjacent au deuxième élément de fixation (11), autour d'une partie corporelle du patient,
- attache de la bande de fixation (6) au deuxième pansement (4) par une fermeture velcro qui est formée par la zone ayant une fonction de velcro de la bande de fixation (6) et la zone ayant une fonction de velcro du deuxième pansement (4),
- dans la mesure où la bande de fixation (6) se trouve dans un état ouvert dans lequel elle a certes été passée autour de la partie corporelle du patient, mais peut encore s'ouvrir d'elle-même : transfert de la bande de fixation (6) de l'état ouvert à un état fermé dans lequel elle ne peut plus s'ouvrir d'elle-même par exemple en formant une fermeture velcro,
- attache d'un premier élément de fixation (5) à deux lumières du tube de cathéter à double lumière à fixer (2), en ce que le premier élément de fixation (5) forme une boucle fermée par une fermeture velcro autour des deux lumières, dans lequel toute la surface tournée vers l'extérieur du premier élément de fixation (5) est configurée comme une zone ayant une fonction de velcro, pour permettre une fixation particulièrement simple et indépendante de la position du premier élément de fixation (5) sur le premier pansement (3),
- attache du premier élément de fixation (5) avec les deux lumières du tube de cathéter à double lumière à fixer (2) au premier pansement (3) par une fermeture velcro entre la zone ayant une fonction de velcro du premier élément de fixation (5) et la zone ayant une fonction de velcro (7) du premier pansement (3),
- attache d'une première lumière du tube de cathéter à double lumière à fixer (2) au deuxième élément de fixation (11) et
- attache d'une deuxième lumière du tube de cathéter à double lumière à fixer (2) au troisième élément de fixation (12), dans lequel la première lumière et la deuxième lumière sont conduites séparément au travers du deuxième élément de fixation (11) et du troisième élément de fixation (12).
